# EUROPEAN PATENT APPLICATION

(11) **EP 4 782 540 A1**
(43) Date of publication of application: **29.07.2026**
(21) Application number: 24868262.7
(22) Date of filing: 18.09.2024
(51) Int. Cl.: C12N 15/113, A61K 31/713, A61K 31/7105, A61K 48/00, A61P 25/00, C12N 1/15, C12N 1/19, C12N 1/21, C12N 5/10, C12N 15/63

(54) **SINGLE-STRANDED POLYNUCLEOTIDE**

(30) Priority: 19.09.2023 JP 2023150963
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: KATSUNO, Masahisa, Nagoya-shi, Aichi 464-8601 (JP); HIRUNAGI, Tomoki, Nagoya-shi, Aichi 464-8601 (JP); SAHASHI, Kentaro, Nagoya-shi, Aichi 464-8601 (JP)
(74) Representative: Vossius & Partner Patentanwälte Rechtsanwälte mbB
(86) International application number: PCT/JP2024/033177
(87) International publication number: WO 2025/063185

(57) **Abstract**

The invention provides a polynucleotide having an exon-skipping effect on SNCA exon 3. Provided is a single-stranded polynucleotide comprising a base sequence A complementary to at least a part of an SNCA gene sequence, wherein the base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGIGC) .

## Description

### Technical Field

The present invention relates to a single-stranded polynucleotide and the like.

### Background Art

Parkinson's disease causes motor symptoms including akinesia, rigidity, and tremor, as well as non-motor symptoms including mental and cognitive dysfunctions and autonomic dysfunction, due to degeneration of dopaminergic neurons in the substantia nigra of the midbrain. The number of patients in Japan is as high as 150,000 to 200,000, and most cases are sporadic neurodegenerative diseases. Treatments include symptomatic therapies, such as dopamine replacement and deep brain stimulation; however, no radical therapeutic method leading to the suppression of neurodegeneration has been created. On the other hand, the core pathology is understood to be the formation of aggregates of α-synuclein protein (SNCA), a component of Lewy bodies, which are a neuropathological finding; thus, research and development of antisense oligonucleotides (ASOs) targeting the mRNA of the SNCA gene are underway. However, gapmer-type ASOs having knockdown potency cannot ensure a sufficient duration of pharmacological effect due to their structure, requiring frequent administration (NPL 1 and 2). In diseases requiring long-term administration, such as Parkinson's disease, the risk of adverse events related to lumbar puncture is a concern. Furthermore, SNCA is considered to be involved in the regulation of synaptic function, and it is assumed that SNCA knockdown will result in inhibition of neurophysiological function.

### Citation List

### Non-patent Literature

NPL 1: Tabrizi S. J., et al. N. Engl. J. Med. 2019; 380: 2307-2316.
NPL 2: Miller T. M., et al. N. Engl. J. Med. 2022; 387: 1099-1110.

### Summary of Invention

### Technical Problem

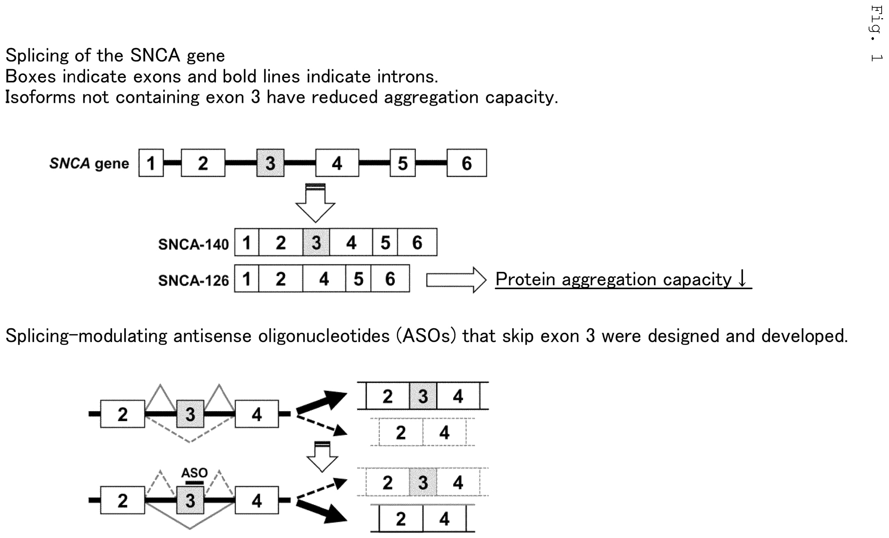

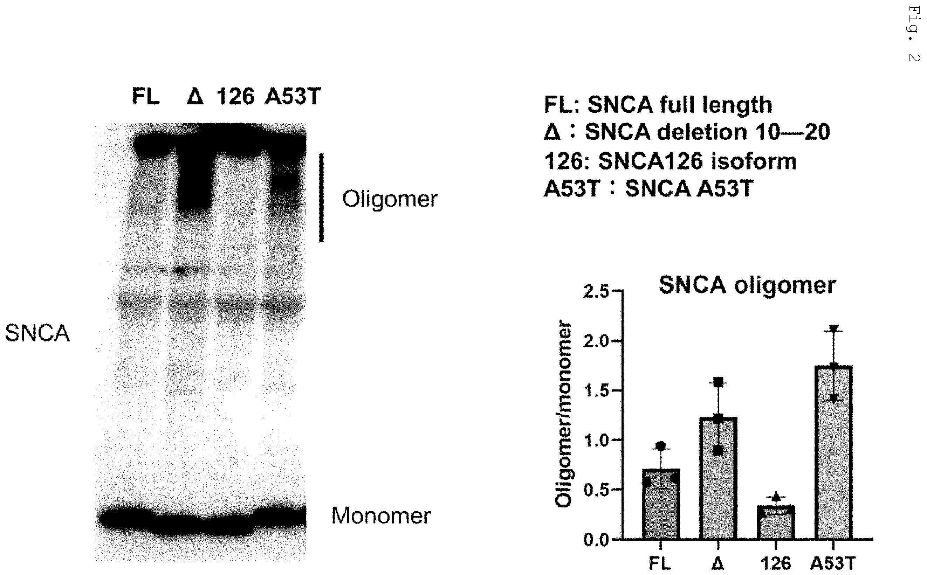

In the course of conducting research, the present inventors focused on exon skipping using an antisense oligonucleotide. An isoform that does not contain SNCA exon 3 does not exhibit aggregative properties that cause synucleinopathies (Test Example 1, and Figs. 1 and 2).

An object of the present invention is to provide a polynucleotide having an exon-skipping effect on SNCA exon 3.

### Solution to Problem

In the course of conducting research, the present inventors focused on the fact that skipping of SNCA exon 3 is possible using a single-stranded polynucleotide. Based on this fact, the present inventors conducted further research and found that the above problem can be solved by a single-stranded polynucleotide comprising a base sequence A complementary to at least a part of an SNCA gene sequence, wherein the base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGTGC). As a result of further research based on this finding, the present inventors have completed the present invention. More specifically, the present invention includes the following aspects.

### Item 1.

A single-stranded polynucleotide comprising:
a base sequence A complementary to at least a part of an SNCA gene sequence,
wherein the base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGTGC) .

### Item 2.

The single-stranded polynucleotide according to Item 1, wherein the base sequence A has a length of 16 or more bases.

### Item 3.

The single-stranded polynucleotide according to Item 1 or 2, wherein the base sequence A has a length of 18 or more bases.

### Item 4.

The single-stranded polynucleotide according to any one of Items 1 to 3, wherein the base sequence A comprises the base sequence a1.

### Item 5.

The single-stranded polynucleotide according to Item 4, wherein the base sequence A comprises, as a sequence comprising the base sequence a1, a base sequence a1' complementary to the base sequence set forth in SEQ ID NO: 38 (5'-GCATGGTGTGGCAAC).

### Item 6.

The single-stranded polynucleotide according to Item 4 or 5, wherein the base sequence A comprises, as a sequence comprising the base sequence a1, a base sequence a11 complementary to a base sequence having a length of 18 or more bases within the base sequence set forth in SEQ ID NO: 3 (5'-GGTGCATGGTGTGGCAACAGGT) .

### Item 7.

The single-stranded polynucleotide according to any one of Items 4 to 6, wherein the base sequence A comprises, as a sequence comprising the base sequence a1, a base sequence a111 complementary to a base sequence having a length of 18 or more bases within the base sequence set forth in SEQ ID NO: 4 (5'-GGTGCATGGTGTGGCAACAGG) .

### Item 8.

The single-stranded polynucleotide according to any one of Items 1 to 7, wherein the base sequence A has a length of 50 or less bases.

### Item 9.

The single-stranded polynucleotide according to any one of Items 1 to 8, comprising a modified nucleotide.

### Item 10.

The single-stranded polynucleotide according to Item 1, consisting of the base sequence set forth in SEQ ID NO: 14 or 32,
wherein in the base sequence, C and T are 2'-O,4'-C-ethylene nucleosides (ENA), G and A are 2'-O-methylated nucleosides (2'OMe), and all internucleoside linkages are phosphorothioate linkages.

### Item 11.

A double-stranded polynucleotide comprising:
an active strand consisting of the single-stranded polynucleotide according to any one of Items 1 to 10; and
a carrier strand complementary thereto.

### Item 12.

A polynucleotide comprising a coding sequence for the single-stranded polynucleotide according to any one of Items 1 to 10.

### Item 13.

The polynucleotide according to Item 12, which is an expression vector for the single-stranded polynucleotide.

### Item 14.

A cell comprising at least one selected from the group consisting of the single-stranded polynucleotide according to any one of Items 1 to 10, the double-stranded polynucleotide according to Item 11, and the polynucleotide according to Item 12 or 13.

### Item 15.

An exon-skipping agent comprising at least one selected from the group consisting of the single-stranded polynucleotide according to any one of Items 1 to 10, the double-stranded polynucleotide according to Item 11, the polynucleotide according to Item 12 or 13, and the cell according to Item 14.

### Item 15A.

A method for skipping SNCA exon 3, comprising administering to a subject at least one selected from the group consisting of the single-stranded polynucleotide according to any one of Items 1 to 10, the double-stranded polynucleotide according to Item 11, the polynucleotide according to Item 12 or 13, and the cell according to Item 14.

### Item 15B.

An agent for use as an exon-skipping agent, comprising at least one selected from the group consisting of the single-stranded polynucleotide according to any one of Items 1 to 10, the double-stranded polynucleotide according to Item 11, the polynucleotide according to Item 12 or 13, and the cell according to Item 14.

### Item 15C.

Use of at least one selected from the group consisting of the single-stranded polynucleotide according to any one of Items 1 to 10, the double-stranded polynucleotide according to Item 11, the polynucleotide according to Item 12 or 13, and the cell according to Item 14 for the manufacture of an exon-skipping agent.

### Item 15D.

Use of at least one selected from the group consisting of the single-stranded polynucleotide according to any one of Items 1 to 10, the double-stranded polynucleotide according to Item 11, the polynucleotide according to Item 12 or 13, and the cell according to Item 14 as an exon-skipping agent.

### Item 16.

A pharmaceutical comprising at least one selected from the group consisting of the single-stranded polynucleotide according to any one of Items 1 to 10, the double-stranded polynucleotide according to Item 11, the polynucleotide according to Item 12 or 13, and the cell according to Item 14.

### Item 17.

The pharmaceutical according to Item 16, which is for preventing or treating synucleinopathy.

### Item 17A.

A method for preventing or treating synucleinopathy, comprising administering to a subject (preferably a subject suffering from synucleinopathy) at least one selected from the group consisting of the single-stranded polynucleotide according to any one of Items 1 to 10, the double-stranded polynucleotide according to Item 11, the polynucleotide according to Item 12 or 13, and the cell according to Item 14.

### Item 17B.

An agent comprising at least one selected from the group consisting of the single-stranded polynucleotide according to any one of Items 1 to 10, the double-stranded polynucleotide according to Item 11, the polynucleotide according to Item 12 or 13, and the cell according to Item 14 for use in the prevention or treatment of synucleinopathy.

### Item 17C.

Use of at least one selected from the group consisting of the single-stranded polynucleotide according to any one of Items 1 to 10, the double-stranded polynucleotide according to Item 11, the polynucleotide according to Item 12 or 13, and the cell according to Item 14 for the manufacture of a pharmaceutical for preventing or treating synucleinopathy.

### Item 17D.

Use of at least one selected from the group consisting of the single-stranded polynucleotide according to any one of Items 1 to 10, the double-stranded polynucleotide according to Item 11, the polynucleotide according to Item 12 or 13, and the cell according to Item 14 as a pharmaceutical for preventing or treating synucleinopathy.

### Item 18.

A reagent comprising at least one selected from the group consisting of the single-stranded polynucleotide according to any one of Items 1 to 10, the double-stranded polynucleotide according to Item 11, the polynucleotide according to Item 12 or 13, and the cell according to Item 14.

### Advantageous Effects of Invention

The present invention can provide a polynucleotide having an exon-skipping effect on SNCA exon 3.

### Brief Description of Drawings

Fig. 1 shows an overview of the present invention.
Fig. 2 shows the results of an α-synuclein (SNCA) aggregation assay using HEK293T cells (Test Example 1). The Western blot results (left) and the ratio of oligomers to monomers quantified by Western blot (right) are shown.
Fig. 3 shows the results of ASO screening using HEK293T cells (Test Example 2). The sequences comprising exon 3 of the SNCA gene and its flanking regions, to which ASOs (ENA #1 to ENA #12) are each complementary (upper), and the RT-PCR results (lower) are shown.
Fig. 4 shows the analysis results (Western blot results) of the effect of ASOs on the SNCA126 protein expression (Test Example 3).
Fig. 5 shows the analysis results of the effect of ASOs on full-length SNCA protein (Test Example 4). The Western blot results (left) and the ratio of SNCA to GAPDH quantified by Western blot (right) are shown.
Fig. 6 shows the analysis results (RT-PCR results) of the effect of ASO chemical modification on skipping efficiency (Test Example 5). The results obtained when HEK293T cells were used (upper) and the results obtained when SH-SY5Y cells were used (lower) are shown.
Fig. 7 shows the results (RT-PCR results) of an exon-skipping test in human iPS-derived dopaminergic neurons (Test Example 6).
Fig. 8 shows the analysis results of the effect on SNCA protein in primary cultured neurons (Test Example 7). The Western blot results (left) and the ratio of SNCA to GAPDH quantified by Western blot (right) are shown.
Fig. 9 shows the analysis results of the effect on the SNCA126 isoform in SNCA E46K mice (Test Example 8). An overview of the primer design (left) and the RT-PCR results (right) are shown.
Fig. 10 shows the analysis results of the effect on human SNCA protein in SNCA E46K mice (Test Example 9). The Western blot results (left) and the ratio of hSNCA to GAPDH quantified by Western blot (right) are shown.
Fig. 11 shows the results of screening sequences comprising ENA #9 and its flanking reigns (Test Example 10). The sequences comprising exon 3 of the SNCA gene and its flanking regions, to which ASOs are each complementary (upper), and the RT-PCR results (lower) are shown.
Fig. 12 shows the results of a comparison of exon-skipping efficiency between ENA #9-2 and ENA #9 (Test Example 11). The RT-PCR results (left) and the ratio of SNCA126 to total SNCA quantified by RT-PCR (right) are shown.
Fig. 13 shows the results (Western blot results) of SNCA126 protein expression induction by ENA #9-2 (Test Example 12).
Fig. 14 shows the effect of ASO on splicing modulation in adult mice (Test Example 13).
Fig. 15 shows the long-term effect of ASO on splicing modulation (Test Example 14).
Fig. 16 shows the long-term effect of ASO on inhibition of SNCA protein (Test Example 15).
Fig. 17 shows the therapeutic effect of ASO in SNCA E46K mice (Test Example 16).

### Description of Embodiments

In the present specification, the expressions "contain" and "comprise" include the concepts of containing, comprising, consisting essentially of, and consisting of.

In the present specification, when an upper limit and a lower limit are separately disclosed for a numeric range, any range defined by any combination of upper and lower limits is also disclosed in the present specification.

### 1. Single-stranded Polynucleotide

In one aspect, the present invention relates to a single-stranded polynucleotide comprising a base sequence A complementary to at least a part of an SNCA gene sequence, wherein the base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGTGC) (also referred to as "the single-stranded polynucleotide of the present invention" in the present specification).

The SNCA gene is not particularly limited as long as it is a human-derived SNCA gene. The entire sequence of the SNCA gene is known. Specifically, the SNCA gene is identified by NCBI Gene ID: 6622, and various sequences thereof (e.g., genomic sequences and mature mRNA sequences) are also registered in the NCBI database.

In the present specification, the SNCA gene sequence refers to a base sequence of the sense strand of the SNCA gene in genomic DNA, and is a base sequence comprising exon sequences and intron sequences.

The phrase "at least a part of the SNCA gene sequence" is not particularly limited as long as a polynucleotide having a complementary sequence can specifically recognize that, and the length is, for example, 14 or more bases. From the viewpoint of reduction of off-target effects, exon-skipping efficiency, and the like, the length is preferably 15 or more bases, more preferably 16 or more bases, even more preferably 17 or more bases, and still more preferably 18 or more bases. The upper limit of the length is not particularly limited and is preferably 50 or less bases, more preferably 40 or less bases, even more preferably 30 or less bases, still more preferably 25 or less bases, and particularly preferably 20 or less bases, for example, from the viewpoint of synthesis efficiency, exon-skipping efficiency, and the like.

Examples of the "at least a part of the SNCA gene sequence" include at least a part of the base sequence set forth in SEQ ID NO: 5 (5'-GTTTTTGTAGGCTCCAAAACCAAGGAGGGAGTGGTGCATGGTGTGGCAACAGGTAAGCTCCATTG TGCTTATATCCAAAGATGATATTTAAAGTATCTAG), which consists of exon 3 and its flanking sequences.

In the present specification, the term "complementary" includes not only complete base complementarity (completely complementary: e.g., A and T or U, and G and C), but also complementarity to the extent that they can hybridize under stringent conditions. The stringent conditions can be determined based on the melting temperature (Tm) of nucleic acids, as taught by Berger and Kimmel (1987, Guide to Molecular Cloning Techniques Methods in Enzymology, Vol. 152, Academic Press, San Diego, CA). Examples of washing conditions after hybridization generally include about "1 × SSC, 0.1% SDS, 37°C." It is preferable that the hybridized state is maintained even after washing under such conditions. Although it is not particularly limited, examples of the washing conditions include more stringent hybridization conditions of about "0.5 × SSC, 0.1% SDS, 42°C," and even more stringent hybridization conditions of about "0.1 × SSC, 0.1% SDS, 65°C."

As stated above, since the complementarity is based on a one-to-one relationship between bases, a base sequence X and a base sequence Y complementary to the base sequence X have the same length of bases.

The base sequence A has a length of, for example, 14 or more bases. From the viewpoint of reduction of off-target effects, exon-skipping efficiency, and the like, the base sequence A has a length of preferably 15 or more bases, more preferably 16 or more bases, still more preferably 17 or more bases, and even more preferably 18 or more bases. The upper limit of the length is not particularly limited and is preferably 50 or less bases, more preferably 40 or less bases, still more preferably 30 or less bases, even more preferably 25 or less bases, and particularly preferably 20 or less bases, for example, from the viewpoint of synthesis efficiency, exon-skipping efficiency, and the like.

The base sequence A is a base sequence having an identity of, for example, 85% or more, preferably 90% or more, more preferably 95% or more, even more preferably 98% or more, still even more preferably 99% or more, and particularly preferably 100% to the base sequence completely complementary to at least a part of the SNCA gene sequence.

In the present specification, the term "identity" of base sequences refers to the degree to which two or more contrastable base sequences match each other. Thus, the higher the degree of match between two base sequences, the higher the identity or similarity of those sequences. The level of base sequence identity is determined, for example, by using FASTA, which is a tool for sequence analysis, with default parameters. Alternatively, the level of base sequence identity can be determined by using the BLAST algorithm by Karlin and Altschul (Karlin S., Altschul S. F. "Methods for assessing the statistical significance of molecular sequence features by using general scoring schemes," Proc. Natl. Acad. Sci. USA. 87: 2264-2268 (1990); Karlin S., Altschul S. F. "Applications and statistics for multiple high-scoring segments in molecular sequences." Proc. Natl. Acad. Sci. USA. 90: 5873-7 (1993)). A program called "BLASTX" based on this BLAST algorithm has been developed. The specific techniques of these analysis methods are known and can be found on the website of the National Center of Biotechnology Information (NCBI) (http://www.ncbi.nlm.nih.gov/).

The base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGTGC).

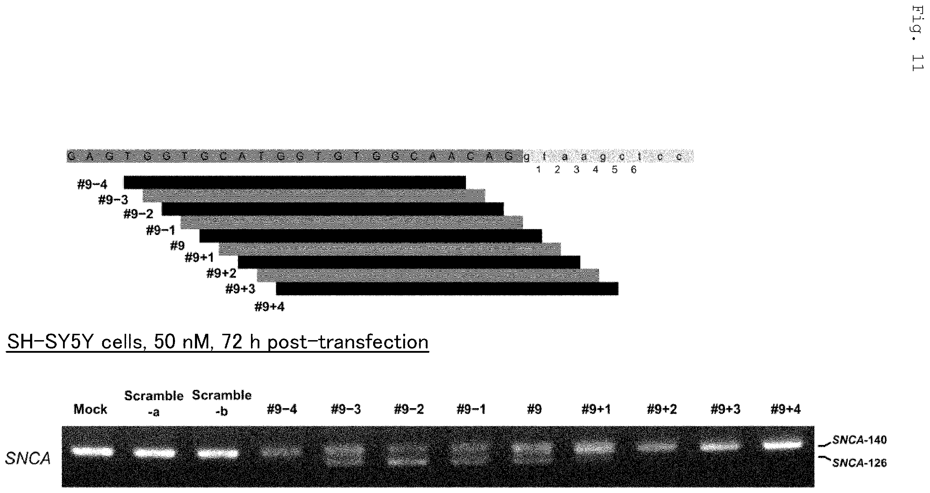

The base sequence set forth in SEQ ID NO: 1 (5'-CATGGTGTGGCAAC) is an overlapping portion of the SNCA gene sequence to which the single-stranded polynucleotides (#9-3, #9-2, #9-1, #9, #9+1) are complementary (see Fig. 11). All of these single-stranded polynucleotides exhibit an exon-skipping effect (see Test Example 10 and Fig. 11), and thus can exert an exon-skipping effect by targeting the overlapping portion of SEQ ID NO: 1.

From the viewpoint of exon-skipping efficiency, the base sequence A preferably comprises, as a sequence comprising the base sequence a1, a base sequence a1' complementary to the base sequence set forth in SEQ ID NO: 38 (5'-GCATGGTGTGGCAAC). The base sequence set forth in SEQ ID NO: 38 (5'-GCATGGTGTGGCAAC) is an overlapping portion of the SNCA gene sequence to which the single-stranded polynucleotides (#9-3, #9-2, #9-1, and #9) are complementary (see Fig. 11). These single-stranded polynucleotides exhibit a greater exon-skipping effect (see Test Example 10 and Fig. 11), and thus can exert a greater exon-skipping effect by targeting the overlapping portion of SEQ ID NO: 38.

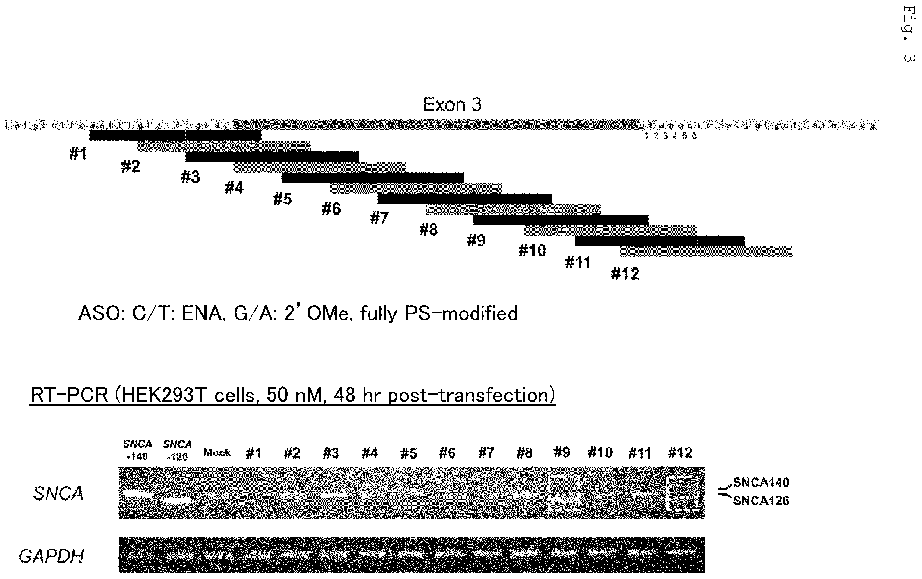

The base sequence set forth in SEQ ID NO: 2 (5'-AGGTAAGCTCCATTGTGC) is a part of the SNCA gene sequence, to which a single-stranded polynucleotide (#12) is complementary (see Fig. 3). This single-stranded polynucleotide exhibits an exon-skipping effect (see Test Example 2 and Fig. 3), and thus can exert an exon-skipping effect by targeting SEQ ID NO: 2.

The base sequence a1 is a base sequence having an identity of, for example, 78% or more, preferably 85% or more, more preferably 92% or more, and particularly preferably 100% to the base sequence completely complementary to the base sequence set forth in SEQ ID NO: 1.

The base sequence a2 is a base sequence having an identity of, for example, 83% or more, preferably 88% or more, more preferably 94% or more, and particularly preferably 100% to the base sequence completely complementary to the base sequence set forth in SEQ ID NO: 2.

The base sequence A preferably comprises the base sequence a1 from the viewpoint of exon-skipping efficiency.

From the viewpoint of exon-skipping efficiency, the base sequence A preferably comprises, as a sequence comprising the base sequence a1, a base sequence a11 complementary to a base sequence having a length of 18 or more bases within the base sequence set forth in SEQ ID NO: 3 (5'-GGTGCATGGTGTGGCAACAGGT), and particularly preferably a base sequence a111 complementary to a base sequence having a length of 18 or more bases within the base sequence set forth in SEQ ID NO: 4 (5'-GGTGCATGGTGTGGCAACAGG) .

A preferable specific example of the base sequence A is a base sequence comprising a base sequence having an identity of, for example, 83% or more, preferably 88% or more, more preferably 94% or more, and particularly preferably 100% to the base sequence set forth in any of SEQ ID NOs: 14, 17, and 31 to 34 (preferably SEQ ID NOs: 14 and 31 to 34, more preferably SEQ ID NOs: 14 and 31 to 33, and particularly preferably SEQ ID NO: 32).

The single-stranded polynucleotide of the present invention may be one in which another base sequence is added to one or both ends of the base sequence A. The length of bases in the base sequence A based on the total length of bases in the single-stranded polynucleotide of the present invention taken as 100% is preferably 50% or more, more preferably 60% or more, still more preferably 70% or more, even more preferably 80% or more, still even more preferably 90% or more, and particularly preferably 100%.

The polynucleotide can be DNA, RNA, or the like, and can comprise a modified nucleoside and a modified internucleotide linkage based thereon.

Examples of modified nucleosides include molecules in which a ribonucleoside, a deoxyribonucleoside, RNA, or DNA is modified so as to improve or stabilize nuclease resistance, increase affinity for a complementary nucleic acid strand, increase cell permeability, or enable visualization, as compared with RNA or DNA. Examples of such molecules include sugar-modified nucleosides, such as 2'-OMe, 2'-MOE, LNA, ENA, AmNA, GuNA, and scpBNA. The polynucleotide may comprise, for example, a modified nucleic acid molecule disclosed in Khvorova & Watts (Nature Biotechnology 35, 238-248 (2017) doi: 10.1038/nbt.3765).

A modified sugar refers to a sugar having any substitution and/or any change compared to a natural sugar moiety (i.e., a sugar moiety found in DNA (2'-H) or RNA (2'-OH)). A sugar-modified nucleoside refers to a modified nucleoside containing a modified sugar. The sugar-modified nucleoside may be any nucleoside in which all or part of the chemical structure of the sugar moiety is modified by addition or substitution of any chemical moiety. Examples include a modified nucleoside substituted with 2'-O-methylribose, a modified nucleoside substituted with 2'-O-propylribose, a modified nucleoside substituted with 2'-methoxyethoxyribose, a modified nucleoside substituted with 2'-O-methoxyethylribose, a modified nucleoside substituted with 2'-O-[2-(guanidinium)ethyl]ribose, and a modified nucleoside substituted with 2'-O-fluororibose; and bridged nucleic acids (BNA) having two cyclic structures obtained by introducing a bridged structure into the sugar moiety, such as a locked nucleic acid (LNA), in which the oxygen atom at the 2'-position and the carbon atom at the 4'-position are bridged via methylene, and an ethylene bridged nucleic acid (ENA) (Nucleic Acids Research, 32, e175 (2004)). Examples further include a peptide nucleic acid (PNA) (Acc. Chem. Res., 32, 624 (1999)), an oxypeptide nucleic acid (OPNA) (J. Am. Chem. Soc., 123, 4653 (2001)), and a peptide ribonucleic acid (PRNA) (J. Am. Chem. Soc., 122, 6900 (2000)).

2'-O-Methyl (2'-OMe) modification of RNA (2'-OMe-RNA) is a modification that occurs naturally as well, improves the binding affinity and nuclease resistance of the modified oligonucleotide, and reduces immunostimulatory properties. 2'-O-Methoxyethyl (2'-MOE) modification confers even greater nuclease resistance than 2'-OMe modification and significantly increases the binding affinity (ΔTm) of the modified nucleotide. 2'-Fluoro (2'-F) modification of RNA (2'-F-RNA) can also be used to increase the affinity of the oligonucleotide. Examples of other 2'-modified nucleic acids include 2'-F-ANA and the 2'-modified derivatives by Sekine et al. (JP5194256B and JP2015-020994A).

A locked nucleic acid (LNA), in which the 2' oxygen and 4' carbon of the ribose are linked, provides a significant increase in binding affinity. In LNA, the 2' oxygen and 4' carbon of the ribose sugar of RNA are fixed in a ring structure. This modification increases specificity and affinity, extends the half-life, and enables effective delivery to a target tissue with lower toxicity. However, oligomers longer than about 8 nucleotides that are fully modified with LNA are known to tend to aggregate, and typically, they are used in a mixture with DNA or other sugar-modified nucleic acids. cEt, which is a methylated analog of LNA, is also as useful as LNA. Tricyclo-DNA (tcDNA) is a constrained nucleotide based on a tricyclic backbone.

Other modified nucleosides that may be used include molecules in which an atom (e.g., a hydrogen atom, an oxygen atom) or a functional group (e.g., a hydroxyl group, an amino group) of a base moiety of a nucleic acid is substituted with another atom (e.g., a hydrogen atom, a sulfur atom), a functional group (e.g., an amino group), or an alkyl group having 1 to 6 carbon atoms, or protected with a protecting group (e.g., a methyl group or an acyl group); molecules in which another chemical substance, such as a lipid, a phospholipid, phenazine, folate, phenanthridine, anthraquinone, acridine, fluorescein, rhodamine, coumarin, or a dye, is added to a nucleoside; and the like.

Particularly preferable examples of modified nucleosides include ENA and 2'-MOE, with ENA being particularly preferred.

The number of modified nucleotides based on the number of nucleosides in the single-stranded polynucleotide of the present invention taken as 100% is not particularly limited and is, for example, 20% or more, preferably 50% or more, more preferably 60% or more, still more preferably 70% or more, even more preferably 80% or more, and particularly preferably 90% or more. In one embodiment of the present invention, all nucleotides may be modified nucleotides.

The term "modified internucleoside linkage" refers to an internucleoside linkage having any substitution or any change compared to a naturally occurring internucleoside linkage (i.e., a phosphodiester linkage). The modified internucleoside linkages include internucleoside linkages containing a phosphorus atom and internucleoside linkages not containing a phosphorus atom. The modified internucleoside linkage may also be one in which any chemical substance is added or substituted on part or all of the chemical structure of the nucleotide phosphodiester bond. Examples include a modified internucleoside linkage substituted with a phosphorothioate linkage and a modified internucleoside linkage substituted with an N3'-P5' phosphoramidate linkage. Other modified internucleoside linkages include (SC5'Rp)-α,β-CNA, PMO, and the like.

Examples of typical phosphorus-containing internucleoside linkages include a phosphodiester linkage, a phosphorothioate linkage (also referred to as a "thiophosphate linkage"), a phosphorodithioate linkage, a phosphotriester linkage, and a methylphosphonate linkage, a methylthiophosphonate linkage, a boranophosphate linkage, and a phosphoramidate linkage.

The modified internucleotide linkage is particularly preferably a phosphorothioate linkage.

The number of modified internucleotide linkages based on the number of internucleotide linkages in the single-stranded polynucleotide of the present invention taken as 100% is not particularly limited, and is, for example, 20% or more, preferably 50% or more, more preferably 60% or more, even more preferably 70% or more, still more preferably 80% or more, and particularly preferably 90% or more. In one embodiment of the present invention, all internucleotide linkages may be modified internucleotide linkages (e.g., phosphorothioate linkages).

In one preferable aspect of the present invention, the single-stranded polynucleotide of the present invention comprises ENA as the modified nucleoside (more preferably ENA and 2'-MOE), and comprises a phosphorothioate linkage as the internucleoside linkage. In this aspect, the number of the modified nucleotides (ENA, or a total of ENA and 2'-MOE) based on the number of nucleosides taken as 100% is, for example, 20% or more, preferably 50% or more, more preferably 60% or more, even more preferably 70% or more, still more preferably 80% or more, particularly preferably 90% or more, and especially preferably 100%. Further, the number of the modified internucleotide linkages (phosphorothioate linkages) based on the number of internucleotide linkages taken as 100% is, for example, 20% or more, preferably 50% or more, more preferably 60% or more, even more preferably 70% or more, still more preferably 80% or more, particularly preferably 90% or more, and especially preferably 100%. In this aspect, the base sequence A is particularly preferably any of SEQ ID NO: 14, 17, or 31 to 34 (particularly preferably SEQ ID NO: 14 or 32).

In the present specification, the bases that constitute the polynucleotide include not only typical bases found in RNA, DNA, and other naturally occurring nucleic acids (e.g., adenine (A), thymine (T), uracil (U), guanine (G), and cytosine (C)), but also other bases such as hypoxanthine (I) and modified bases. Examples of modified bases include pseudouracil, 3-methyluracil, dihydrouracil, 5-alkylcytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methoxyuracil, 5-methyl-2-thiouracil, 2-methylthio-N6-isopentenyladenine, uracil-5-oxyacetic acid, 2-thiocytosine, purine, 2-aminopurine, isoguanine, indole, imidazole, xanthine, and cyanuric acid.

In one aspect, the present invention relates to a double-stranded polynucleotide comprising an active strand consisting of the single-stranded polynucleotide of the present invention and a carrier strand complementary thereto (also referred to in the present specification as "the double-stranded polynucleotide of the present invention").

The double-stranded polynucleotide of the present invention may be a heteroduplex oligonucleotide (also referred to as "HDO"). HDO is an artificial functional nucleic acid composed of a double strand of an active strand and a carrier strand complementary thereto (see, for example, Nishina et al., Nature Communications, Vol. 6, Article number: 7969 (2015), JP6112569B, JP6638923B, JP6416301B, and JP6604544B). An active strand (also referred to as a "first strand") and a carrier strand complementary to the active strand (also referred to as a "second strand") may be contained. The nucleotide length of the carrier strand is not particularly limited and is typically about 13 to 30 bases, for example, 13 bases, 14 bases, 15 bases, 16 bases, 17 bases, 18 bases, 19 bases, 20 bases, 21 bases, or 22 bases. The nucleotide lengths of the active strand and the carrier strand are not necessarily the same. For example, the carrier strand may be shorter than the active strand, or conversely, may be longer. The carrier strand is also not necessarily completely complementary to the active strand, and may contain mismatches or bulges. The complementarity may be, for example, 90% or more or 95% or more. While not intending to be bound by any particular theory, it is assumed that after HDO is delivered into a cell, the active strand and the carrier strand separate, and the active strand functions as the single-stranded polynucleotide of the present invention within the cell.

In one aspect, the present invention relates to a polynucleotide comprising a coding sequence for the single-stranded polynucleotide of the present invention (also referred to in the present specification as "the polynucleotide of the present invention").

The coding sequence is not particularly limited as long as it is a base sequence that produces the single-stranded polynucleotide of the present invention when transcribed by an RNA polymerase.

The polynucleotide of the present invention is, for example, DNA, and can be linear or circular.

The polynucleotide of the present invention may be an expression vector for the single-stranded polynucleotide of the present invention.

Typical examples of the polynucleotide of the present invention include a promoter and a coding sequence for the single-stranded polynucleotide of the present invention placed under control of the promoter.

The promoter is not particularly limited, and a pol II promoter can be used; however, from the viewpoint of performing more accurate transcription of relatively short RNAs, a pol III promoter is preferred. Examples of pol III promoters include, but are not particularly limited to, mouse and human U6-snRNA promoters, a human H1-RNase P RNA promoter, and a human valine-tRNA promoter.

The polynucleotide of the present invention may further comprise other elements (e.g., a multiple cloning site (MCS), a drug resistance gene, and a replication origin), as necessary. Examples of drug resistance genes include a chloramphenicol resistance gene, a tetracycline resistance gene, a neomycin resistance gene, an erythromycin resistance gene, a spectinomycin resistance gene, a kanamycin resistance gene, a hygromycin resistance gene, and a puromycin resistance gene. The type of vector is not particularly limited, and examples include plasmid vectors, such as expression plasmids for animal cells; and viral vectors, such as retroviruses, lentiviruses, adenoviruses, adeno-associated viruses, herpesviruses, and Sendai viruses.

The single-stranded polynucleotide of the present invention, the double-stranded polynucleotide of the present invention, and the polynucleotide of the present invention can be easily produced according to known genetic engineering techniques. For example, chemical synthesis, PCR, restriction enzyme cleavage, a DNA ligation technique, and the like may be used for production.

### 2. Cell

In one aspect, the present invention relates to a cell comprising at least one selected from the group consisting of the single-stranded polynucleotide of the present invention, the double-stranded polynucleotide of the present invention, and the polynucleotide of the present invention (sometimes referred to as "the cell of the present invention" in the present specification).

The cell of the present invention may be obtained, for example, by introducing at least one selected from the group consisting of the single-stranded polynucleotide of the present invention, the double-stranded polynucleotide of the present invention, and the polynucleotide of the present invention.

The cell of the present invention is preferably an isolated cell. The cell of the present invention may be a cultured cell, and may be a primary cultured cell or a passaged cell. The cell of the present invention may be a cell line. The cell may be an iPS cell.

Examples of the cells of the present invention include *Escherichia coli,* such as *Escherichia coli* K12; *Bacillus* bacteria, such as *Bacillus subtilis* MI114; yeasts, such as *Saccharomyces cerevisiae* AH22; and insect cells or animal cells, such as the Sf cell line derived from *Spodoptera frugiperda,* the High Five cell line derived from *Trichoplusia ni,* and olfactory neurons. Preferable examples of animal cells include cultured cells derived from mammals, such as COS7 cells, CHO cells, HEK293 cells, HEK293FT cells, HeLa cells, PC12 cells, N1E-115 cells, and SH-SY5Y cells.

### 3. Application

The single-stranded polynucleotide of the present invention, the double-stranded polynucleotide of the present invention, the polynucleotide of the present invention, and the cell of the present invention (these may be collectively referred to below as "the active ingredient") can be used, for example, for pharmaceuticals, reagents, and the like (these may be referred to as "the therapeutic agent of the present invention" in the present specification), and, for example, for exon-skipping agents and agents for preventing or treating synucleinopathies.

Specifically, exon skipping refers to skipping of exon 3 of the SNCA gene/mRNA. This allows for the expression of exon 3-deleted α-synuclein mRNA/protein while reducing full-length α-synuclein mRNA/protein. This approach has an advantage in that suppressing the formation of aggregates is possible while maintaining the physiological function of α-synuclein because the α-synuclein isoform is preserved, unlike conventional knockdown.

Synucleinopathy is a neurodegenerative disease pathologically characterized by the formation and accumulation of α-synuclein protein aggregates. Examples of synucleinopathy include Parkinson's disease, dementia with Lewy bodies, pure autonomic failure, and multiple system atrophy.

The therapeutic agent of the present invention is not particularly limited as long as it comprises the active ingredient, and additives may be further contained, if necessary. Examples of additives include bases, carriers, solvents, dispersants, emulsifiers, buffers, stabilizers, excipients, binders, disintegrants, lubricants, thickeners, moisturizers, colorants, fragrances, and chelating agents.

The usage of the therapeutic agent of the present invention is not particularly limited, and a suitable usage can be adopted depending on the type of therapeutic agent. The therapeutic agent of the present invention can be used, for example, in vitro (e.g., added to the medium of cultured cells) or in vivo (e.g., administered to an animal) depending on the purpose of use thereof.

The target for applying the therapeutic agent of the present invention is not particularly limited. In mammals, examples include humans, monkeys, mice, rats, dogs, cats, rabbits, pigs, horses, cows, sheep, goats, and deer. Cells for target include animal cells. There is no particular limitation on the type of cells, and examples include blood cells, hematopoietic stem cells, progenitor cells, gametes (sperm and eggs), fibroblasts, epithelial cells, vascular endothelial cells, nerve cells, liver cells, keratinocytes, muscle cells, epidermal cells, endocrine cells, ES cells, iPS cells, tissue stem cells, and cancer cells.

The therapeutic agent of the present invention can be in any dosage form, such as an oral dosage form, such as tablets (including orally disintegrating tablets, chewable tablets, effervescent tablets, troches, jelly drops, etc.), pills, granules, fine granules, powders, hard capsules, soft capsules, dry syrup, liquids (including drinks, suspensions, and syrup), and jellies; or a parenteral dosage form, such as injectable preparations (e.g., drip infusions (e.g., preparations for intravenous drip infusion), intravenous injections, intramuscular injections, subcutaneous injections, and intradermal injections), external preparations (e.g., ointments, cataplasms, and lotions), suppositories, inhalants, eye drops, ophthalmic ointments, nasal drops, and ear drops. The active ingredient can be administered in a state of being complexed with particles (e.g., lipid particles or exosomes), or in a state of being encapsulated in the particles.

The administration route for the therapeutic agent of the present invention is not particularly limited as long as desired effects are achieved. Administration routes include oral administration, tube feeding, and enteral administration, such as enema administration; and parenteral administration, such as intravenous administration, intra-arterial administration, intramuscular administration, intracardiac administration, subcutaneous administration, intradermal administration, intraperitoneal administration, and intranasal administration.

The content of the active ingredient in the therapeutic agent of the present invention depends on factors such as the mode of use, the target of application, and the condition of the target. The content of the active ingredient in the therapeutic agent of the present invention is not limited and may be, for example, 0.0001 to 100 wt%, and preferably 0.001 to 50 wt%.

The dosage of the therapeutic agent of the present invention when administered to an animal is not particularly limited as long as it is an amount effective to exhibit drug efficacy. Typically, the dosage for oral administration on an active-ingredient weight basis is 0.1 to 1000 mg/kg body weight per day, preferably 0.5 to 500 mg/kg body weight per day, whereas the dosage for parenteral administration on an active-ingredient weight basis is 0.01 to 100 mg/kg body weight per day, and preferably 0.05 to 50 mg/kg body weight per day. The dosage above can be appropriately increased or decreased according to the age, pathological conditions, symptoms, and the like of the animal.

### Examples

The present invention will be described in detail below based on Examples. However, the present invention is not limited by these Examples.

### Test Example 1. α-Synuclein (SNCA) Aggregation Assay using HEK293T Cells

HEK293T cells (24-well plate) were used to overexpress 500 ng of full-length SNCA (FL), a mutant SNCA lacking the amino acids at positions 10 to 20 (Δ), an SNCA 126 isoform lacking exon 3 (126), and an A53T mutant SNCA (A53T). After 48 hours, oligomer formation was evaluated by Western blot. Transfection was performed using Lipofectamine 2000 (Thermo Fisher Scientific Inc.), and 400 ng of α-synuclein fibrotic protein (CSR-SYN03, Cosmo Bio Co., Ltd.) was added to each well as an aggregation promoter.

Fig. 2 shows the results. The results revealed that the SNCA 126 isoform (126), which lacks exon 3, does not exhibit aggregative properties.

### Test Example 2. ASO Screening using HEK293T Cells

Twelve ASOs (ENA #1 to #12) were designed based on exon 3 and its flanking sequences in the SNCA gene (with sequences shifted by 5 bases each), and custom-synthesized by KNC Laboratories Co., Ltd.

In the following ASOs (ENA #1 to #12), C and T are 2'-O,4'-C-ethylene nucleosides (ENA), G and A are 2'-O-methylated nucleosides (2'OMe), and all bases are phosphorothioate (PS)-modified.
ENA #1. AGCCTACAAAAACAAATT (SEQ ID NO: 6)
ENA #2. TTTGGAGCCTACAAAAAC (SEQ ID NO: 7)
ENA #3. TTGGTTTTGGAGCCTACA (SEQ ID NO: 8)
ENA #4: CCTCCTTGGTTTTGGAGC (SEQ ID NO: 9)
ENA #5: CACTCCCTCCTTGGTTTT (SEQ ID NO: 10)
ENA #6: TGCACCACTCCCTCCTTG (SEQ ID NO: 11)
ENA #7: CACCATGCACCACTCCCT (SEQ ID NO: 12)
ENA #8: TGCCACACCATGCACCAC (SEQ ID NO: 13)
ENA #9: CCTGTTGCCACACCATGC (SEQ ID NO: 14)
ENA #10: GCTTACCTGTTGCCACAC (SEQ ID NO: 15)
ENA #11: ATGGAGCTTACCTGTTGC (SEQ ID NO: 16)
ENA #12: GCACAATGGAGCTTACCT (SEQ ID NO: 17).

ENA #1 to ENA #12 were each administered at 50 nM to HEK293T cells using Lipofectamine RNAiMAX (Thermo Fisher Scientific Inc.) as a transfection reagent, and RNA was collected after 48 hours. The collected RNA was reverse-transcribed into cDNA, and isoform changes resulting from exon 3 skipping were analyzed by PCR using primers with the following sequences.

### Primer Sequences

SNCA forward: ATGGATGTATTCATGAAAGGACT (SEQ ID NO: 18);
SNCA reverse: TTAGGCTTCAGGTTCGTAGT (SEQ ID NO: 19);
GAPDH forward: GAGTCAACGGATTTGGTCGT (SEQ ID NO: 20); and
GAPDH reverse: TTGATTTTGGAGGGATCTCG (SEQ ID NO: 21).

Fig. 3 shows the results. The administration of ENA #9 and ENA #12 each resulted in the expression of the SNCA 126 isoform, and administration of ENA #10 and ENA #11 each also resulted in a slight isoform change.

### Test Example 3: Analysis of Effect of ASO on SNCA126 Protein Expression

ENA #6 (control), ENA #9, and ENA #12 were each administered at concentrations of 0, 5, 10, and 50 nM to HEK293T cells using Lipofectamine RNAiMAX, and changes in SNCA protein isoforms after 48 hours were analyzed by Western blot. The antibodies used were anti-SNCA antibody C-20 (Santa Cruz Biotechnology) and anti-GAPDH antibody ab9485 (Abcam).

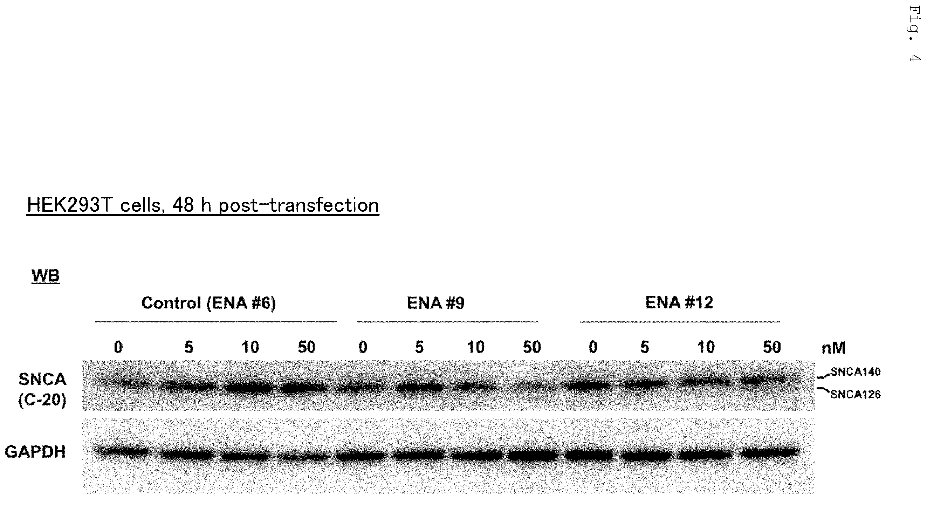

Fig. 4 shows the results. While the administration of ENA #9 and ENA #12 each resulted in the expression of the SNCA 126 isoform resulting from exon skipping, the administration of ENA #6 did not result in the expression of the same isoform.

### Test Example 4: Analysis of Effect of ASO on Full-length SNCA Protein

ENA #6 (control), ENA #9, and ENA #12 were each administered at 50 nM to HEK293T cells using Lipofectamine RNAiMAX, and the expression level of full-length SNCA protein after 72 hours was analyzed by Western blot. The antibodies used were anti-SNCA antibody MJFR1 (Abcam) and anti-GAPDH antibody ab9485 (Abcam).

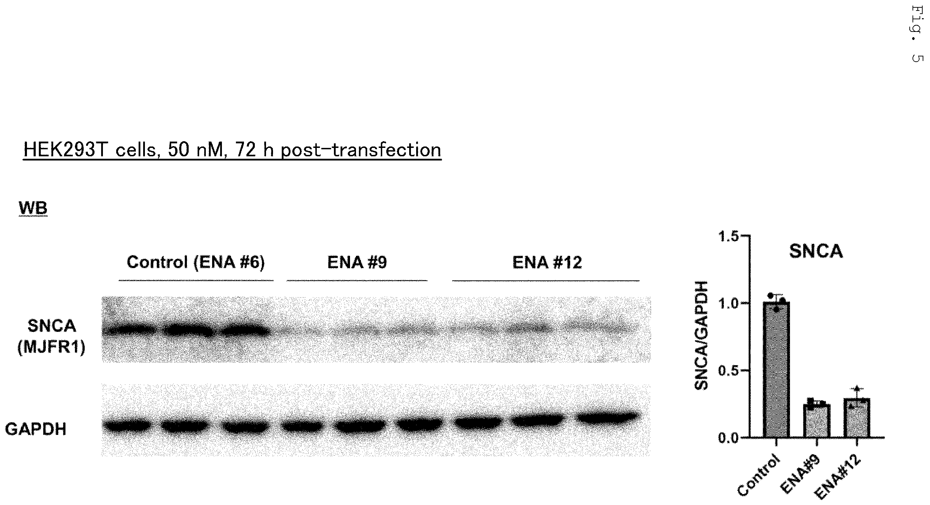

Fig. 5 shows the results. Compared to ENA #6 (control), the administration of ENA #9 and ENA #12 each resulted in a decrease in full-length SNCA protein. Furthermore, unexpectedly, no adverse effects, such as cell death or decreased cell proliferation, were observed.

### Test Example 5: Analysis of Effect of ASO Chemical Modification on Skipping Efficiency

An ASO that has the same sequence as that of ENA #12 and in which all bases were 2'-O-methoxyethyl (MOE)-modified (MOE #12) was designed and custom-synthesized by Hokkaido System Science Co., Ltd.

In the following ASO (MOE #12), all bases were MOE-modified and PS-modified.
MOE #12: GCACAATGGAGCTTACCT (SEQ ID NO: 17).

ENA #9, ENA #12, and MOE #12 were each administered at 50 nM to HEK293T cells using Lipofectamine RNAiMAX, and RNA was collected after 48 hours. Similarly, ENA #6, ENA #9, ENA #12, and MOE #12 were each administered at 50 nM to SH-SY5Y cells using Lipofectamine RNAiMAX, and RNA was collected after 72 hours. The collected RNA was reverse-transcribed into cDNA, and isoform changes resulting from exon 3 skipping were analyzed by PCR using primers with the following sequences.
Primer Sequences (the forward sequence is the same as that used in Test Example 2, while the reverse sequence is different from that used in Test Example 2)
SNCA forward: ATGGATGTATTCATGAAAGGACT (SEQ ID NO: 18); and
SNCA reverse: CCTTCTTCATTCTTGCCCAA (SEQ ID NO: 22).

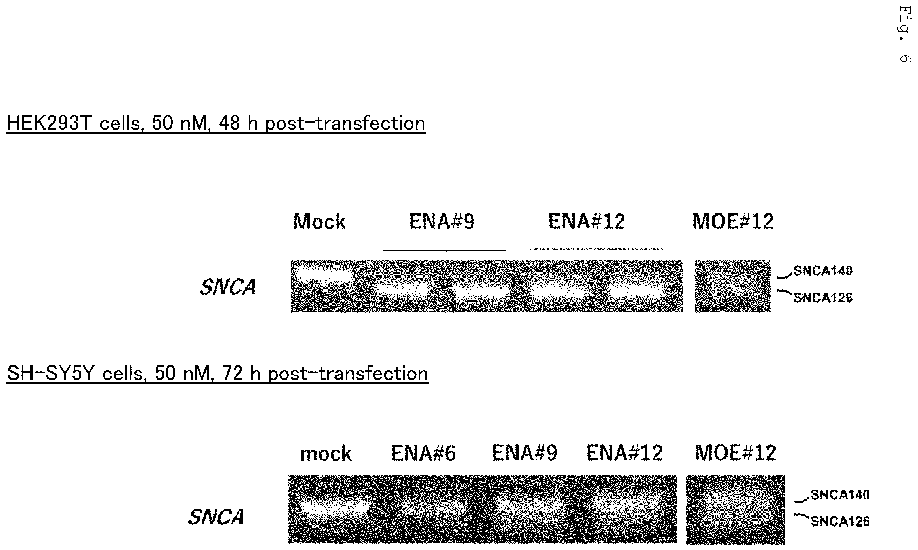

Fig. 6 shows the results. In both HEK293T cells and SH-SY5Y cells, the administration of ENA #9, ENA #12, and MOE #12 each resulted in the expression of the SNCA 126 isoform.

### Test Example 6: Exon-skipping Test in Human iPS Cell-derived Dopaminergic Neurons

ENA #9 and ENA #12 were each administered at 50 nM to iCell (registered trademark) DopaNeurons SNCA A53T (FUJIFILM), which are dopaminergic neurons derived from a Parkinson's disease iPSC model, and RNA was collected 48 hours after the administration.

For transfection, Lipofectamine 2000 Reagent (Thermo Fisher Scientific Inc.) was used at half the volume specified in the manufacturer's protocol (0.5 µl/well in a 24-well plate). The collected RNA was reverse-transcribed into cDNA, and isoform changes resulting from exon 3 skipping were analyzed by PCR using primers with the following sequences.

### Primer Sequences (the same as those used in Test Example 2)

SNCA forward: ATGGATGTATTCATGAAAGGACT (SEQ ID NO: 18); and
SNCA reverse: TTAGGCTTCAGGTTCGTAGT (SEQ ID NO: 19).

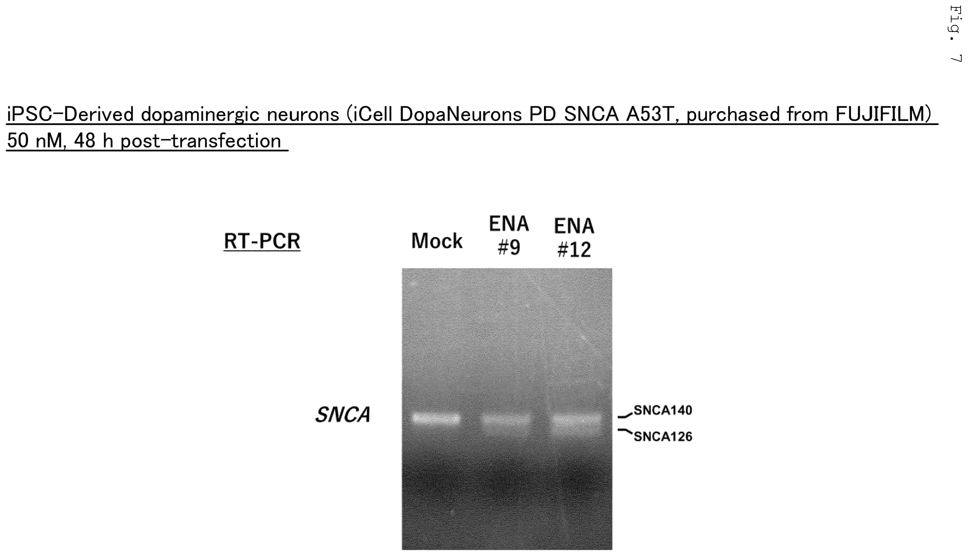

Fig. 7 shows the results. The administration of ENA #9 and ENA #12 each resulted in the expression of the SNCA 126 isoform.

### Test Example 7: Analysis of Effect on SNCA Protein in Primary Cultured Neurons

B6N.Cg-Tg(SNCA*E46K)3Elan/J (Strain No: 018768; referred to below as "the SNCA E46K mice") for expressing the full-length E46K mutant SNCA gene via a bacterial artificial chromosome (BAC) were purchased from The Jackson Laboratory.

Cerebral cortical neurons were collected from the SNCA E46K mice at embryonic day 15 using a neuron dissociation solution (FUJIFILM) and cultured using a neuron culture medium (FUJIFILM). On the second day of culture, the neurons were transfected with 50 nM of ENA #9 using Lipofectamine RNAiMAX (Thermo Fisher Scientific Inc.), and the SNCA protein expression 5 days after transfection was analyzed by Western blot.

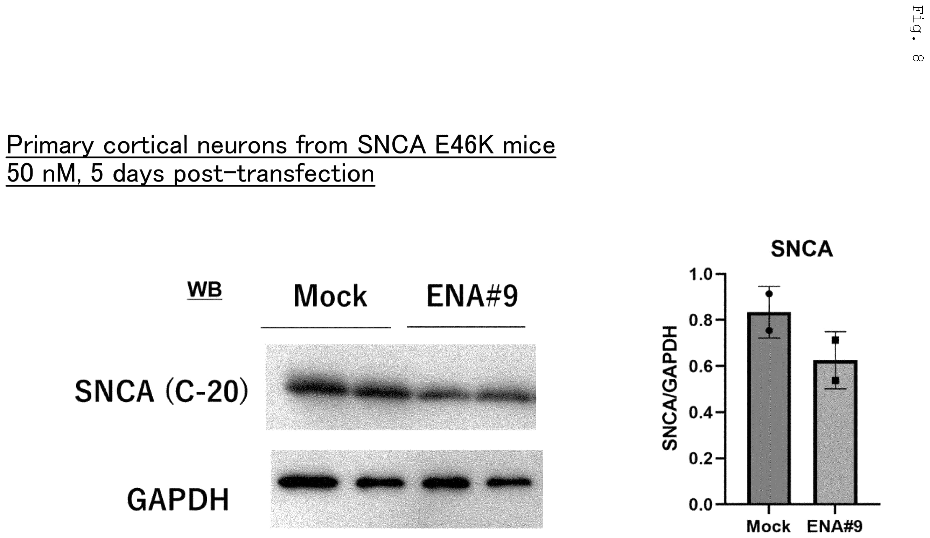

Fig. 8 shows the results. The administration of ENA #9 to the cerebral cortical neurons resulted in a decrease in the full-length SNCA protein expression.

### Test Example 8: Analysis of Effect on SNCA126 Isoform in SNCA E46K Mice

Saline or ENA #9 was administered into the cerebral ventricle of the SNCA E46K mice on postnatal day 2 (P2). The mice were dissected on P11, i.e., nine days after administration, to collect the cerebral cortex, and RNA was extracted. The RNA was reverse-transcribed into cDNA, and the expression of SNCA140 and SNCA126 isoforms was analyzed by PCR using primers with the following sequences. For the PCR, HiDi DNA polymerase (myPOLS Biotec), which is capable of discriminating a single-base difference at the 3' end of the primer, was used.
SNCA 140 forward: AGAGGGTGTTCTCTATGTAGG (G primer) (SEQ ID NO: 23);
SNCA 126 forward: AGAGGGTGTTCTCTATGTAGT (T primer) (SEQ ID NO: 24);
SNCA reverse (for both 140 and 126): CTTCTTCATTCTTGCCCAA (SEQ ID NO: 25);
Gapdh forward: CGTCCCGTAGACAAAATGGT (SEQ ID NO: 26); and
Gapdh reverse: GAATTTGCCGTGAGTGGAGT (SEQ ID NO: 27).

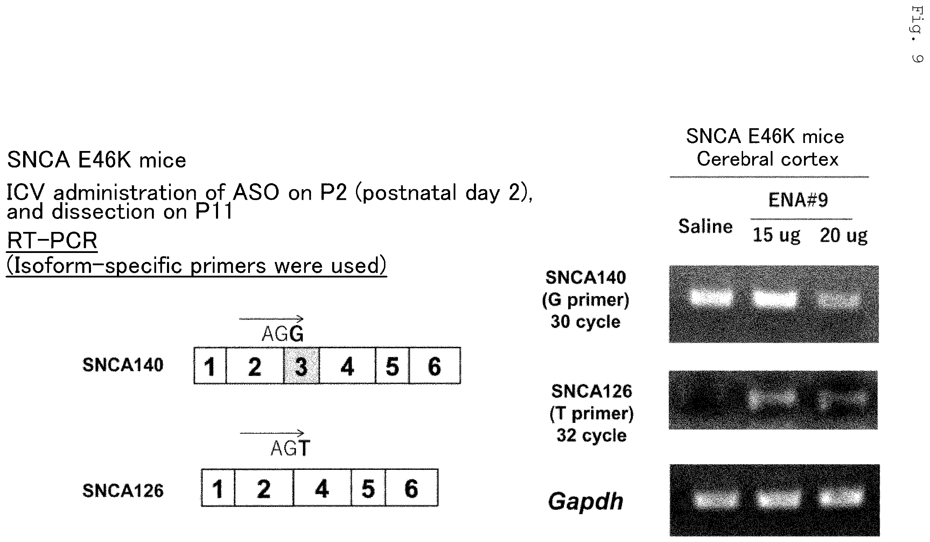

Fig. 9 shows the results. The administration of 15 µg and 20 µg of ENA #9 each resulted in the expression of SNCA 126, whereas the administration of saline resulted in no expression of SNCA 126. Additionally, the administration of 20 µg of ENA #9 resulted in a decrease in the expression level of SNCA 140.

### Test Example 9: Analysis of Effect on Human SNCA Protein in SNCA E46K Mice

ENA #9 (10 µg) was administered into the cerebral ventricle of the SNCA E46K mice on postnatal day 1 (P1). Protein was extracted from the mouse cerebral cortex 6 days after administration (P7), and the human SNCA (hSNCA) protein level was evaluated by Western blot.

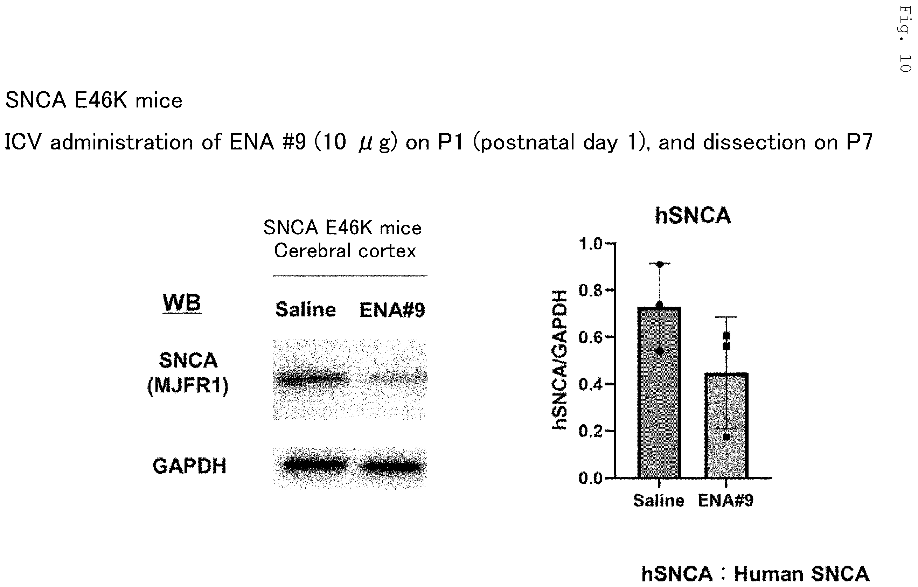

Fig. 10 shows the results. The administration of ENA #9 resulted in a decrease in the hSNCA protein level in the mouse cerebral cortex, compared to the administration of saline.

### Test Example 10: Screening Sequences Flanking ENA #9

In order to analyze the sequences comprising ENA #9 and its flanking regions, eight ASOs, each having a sequence that differs from ENA #9 by one base (items 3 to 6 and items 8 to 11 below), and two ASOs, each composed of a scrambled sequence of ENA #9 as controls (items 1 and 2 below), were designed and custom-synthesized by KNC Laboratories Co., Ltd.

In all of the following ASOs, C and T are 2'-O,4'-C-ethylene nucleosides (ENA), G and A are 2'-O-methylated nucleosides (2'OMe), and all bases are phosphorothioate (PS)-modified.
1. ENA #9 scramble-a. CACTACCAGCTGTTCCGC (SEQ ID NO: 28);
2. ENA #9 scramble-b. ACCCCGGCCTCACTGTTA (SEQ ID NO: 29);
3. ENA #9-4. TTGCCACACCATGCACCA (SEQ ID NO: 30);
4. ENA #9-3. GTTGCCACACCATGCACC (SEQ ID NO: 31);
5. ENA #9-2. TGTTGCCACACCATGCAC (SEQ ID NO: 32);
6. ENA #9-1. CTGTTGCCACACCATGCA (SEQ ID NO: 33);
7. ENA #9. CCTGTTGCCACACCATGC (SEQ ID NO: 14;
8. ENA #9+1. ACCTGTTGCCACACCATG (SEQ ID NO: 34);
9. ENA #9+2. TACCTGTTGCCACACCAT (SEQ ID NO: 35);
10. ENA #9+3. TTACCTGTTGCCACACCA (SEQ ID NO: 36);
11. ENA #9+4. CTTACCTGTTGCCACACC (SEQ ID NO: 37).

The above ASOs (items 1 to 11) were each administered at 50 nM to the SH-SY5Y cells using Lipofectamine RNAiMAX, and RNA was collected after 72 hours. The collected RNA was reverse-transcribed into cDNA, and isoform changes resulting from exon 3 skipping were analyzed by PCR using primers with the following sequences.

### Primer Sequences (the same as those used in Test Example 5)

SNCA forward: ATGGATGTATTCATGAAAGGACT (SEQ ID NO: 18); and
SNCA reverse: CCTTCTTCATTCTTGCCCAA (SEQ ID NO: 22).

Fig. 11 shows the results. The administration of ENA #9-3, #9-2, #9-1, #9, and #9+1 each resulted in the SNCA 126 isoform expression.

### Test Example 11. Comparison of Exon-skipping Efficiency between ENA #9-2 and ENA #9

In the same manner as in Test Example 10, ENA #9 scramble-a, ENA #9-2, and ENA #9 were each administered at 50 nM to the SH-SY5Y cells, and RNA was collected 72 hours after administration. The following primers were used to compare SNCA exon 3-skipping efficiency.

### Primer Sequences (the same as those used in Test Example 5)

SNCA forward: ATGGATGTATTCATGAAAGGACT (SEQ ID NO: 18); and
SNCA reverse: CCTTCTTCATTCTTGCCCAA (SEQ ID NO: 22).

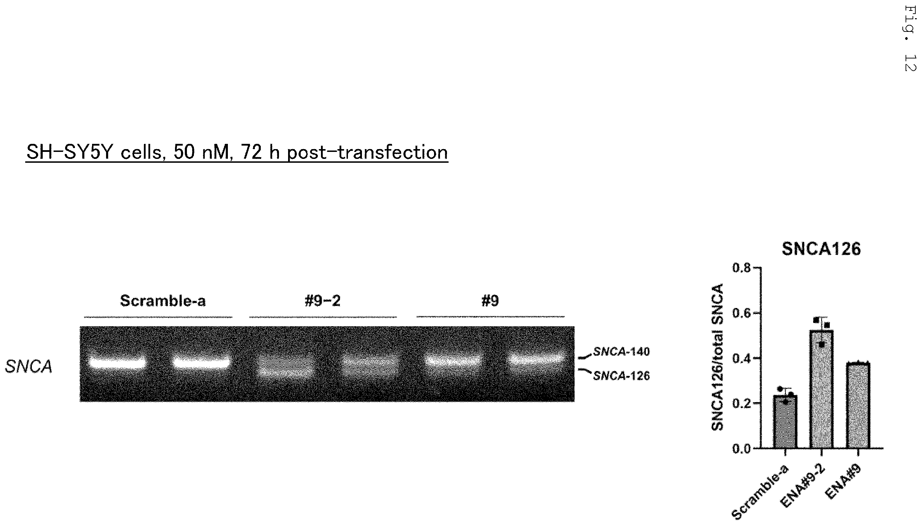

Fig. 12 shows the results. The ratio of SNCA126 (SNCA126/total SNCA) was about 0.2 for ENA #9 scramble-a, about 0.55 for ENA #9-2, and about 0.4 for ENA #9, with ENA #9-2 showing the highest efficiency.

### Test Example 12. Induction of SNCA126 Protein Expression by ENA #9-2

In the same manner as in Test Example 3, scramble-a and ENA #9-2 were each administered at 50 nM to HEK293T cells using Lipofectamine RNAiMAX, and changes in SNCA protein isoforms after 48 hours were analyzed by Western blot. The antibodies used were anti-SNCA antibody C-20 (Santa Cruz Biotechnology) and anti-GAPDH antibody ab9485 (Abcam).

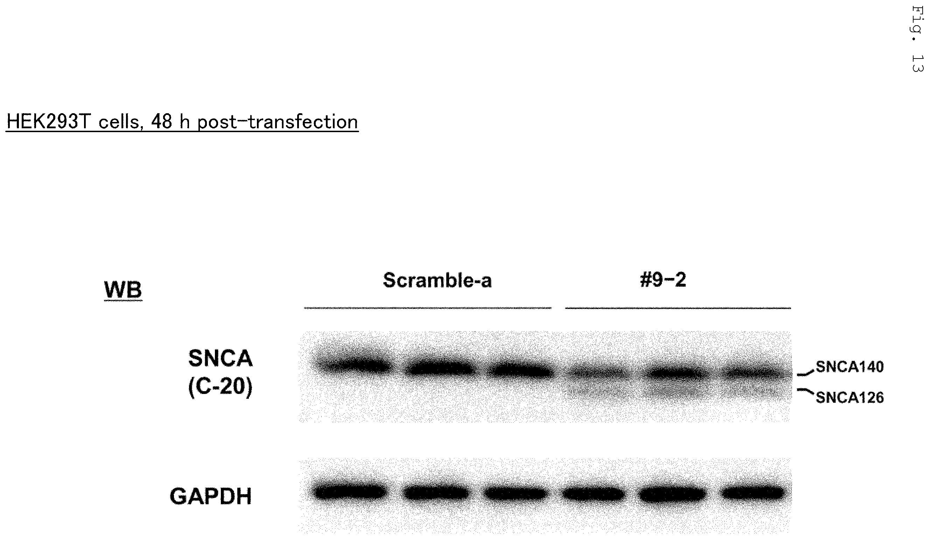

Fig. 13 shows the results. While the administration of ENA #9-2 confirmed the expression of the SNCA 126 isoform resulting from exon skipping, the administration of scramble-a did not result in the expression of the same isoform.

### Test Example 13. Effect of ASO on Splicing Modulation in Adult Mice

ENA #9 was administered into the cerebral ventricle of 11-week-old SNCA E46K mice at doses of 0 (saline), 120, or 150 µg, and the mouse cerebral cortex and midbrain were collected after 2 weeks. RNA was extracted from the collected tissue, and the expression of SNCA140 and SNCA126 isoforms was analyzed by PCR using primers with the following sequences. For the PCR, HiDi DNA polymerase (myPOLS Biotec), which is capable of discriminating a single-base difference at the 3' end of the primer, was used.
SNCA 140 forward: AGAGGGTGTTCTCTATGTAGG (G primer) (SEQ ID NO: 39) ;
SNCA 126 forward: AGAGGGTGTTCTCTATGTAGT (T primer) (SEQ ID NO: 40) ;
SNCA reverse (for both 140 and 126): CTTCTTCATTCTTGCCCAA (SEQ ID NO: 41);
Gapdh forward: CGTCCCGTAGACAAAATGGT (SEQ ID NO: 42); and
Gapdh reverse: GAATTTGCCGTGAGTGGAGT (SEQ ID NO: 43).

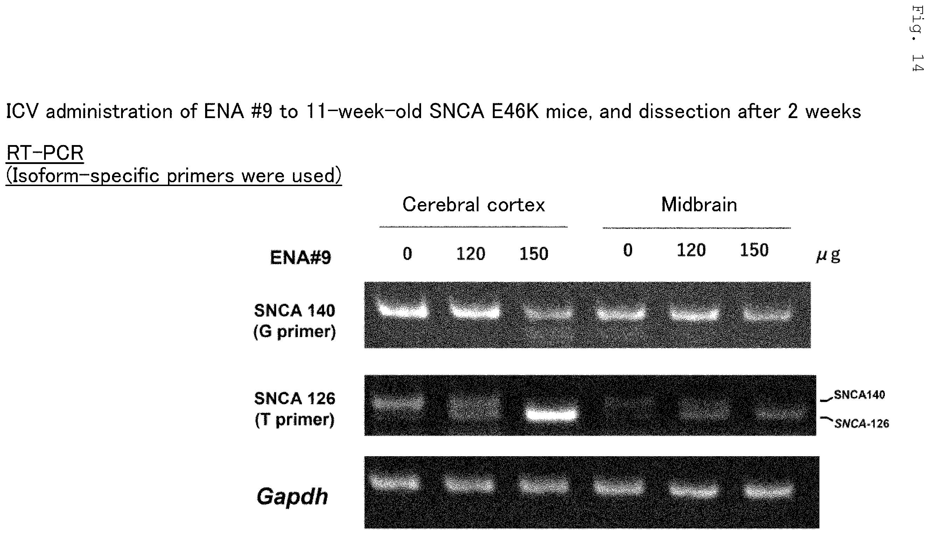

Fig. 14 shows the results. While the administration of 120 µg or 150 µg of ENA #9 each resulted in the expression of SNCA 126, the administration of saline did not result in the expression of SNCA 126. The administration of 150 µg of ENA #9 resulted in a decrease in the SNCA 140 expression level.

### Test Example 14: Long-term Effect of ASO on Splicing Modulation

Saline or 20 µg of ENA #9 was administered into the cerebral ventricle of the SNCA E46K mice on postnatal day 2 (P2), and the mouse cerebral cortex was collected one year after the administration. RNA was extracted from the collected tissue, and the SNCA isoform expression was analyzed in the same manner as in Supplemental Fig. 1.

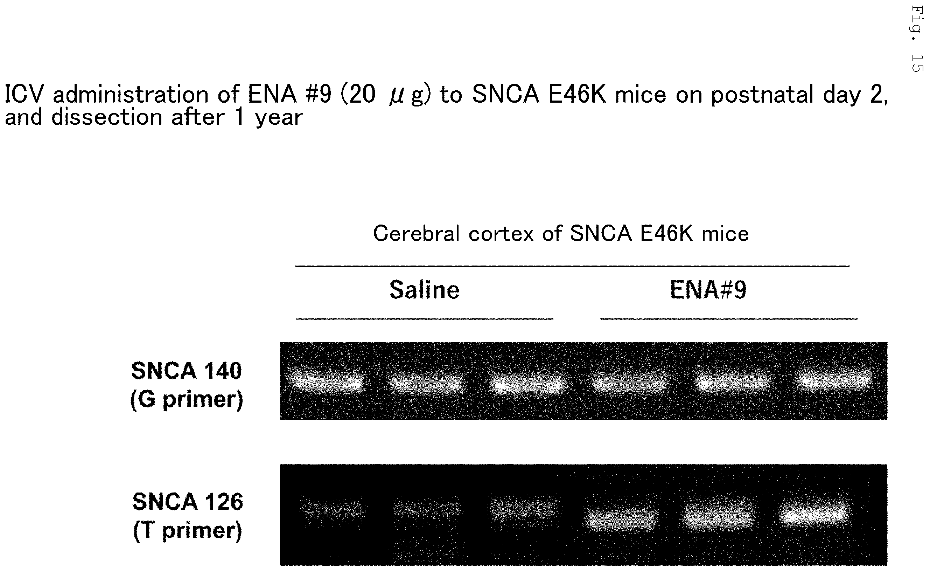

Fig. 15 shows the results. The group to which ENA #9 was administered resulted in the expression of SNCA 126, whereas the group to which saline was administered did not result in the expression of SNCA 126. Further, the human SNCA (hSNCA) protein level was evaluated by Western blot, which revealed a decrease in the hSNCA protein level in the mouse cerebral cortex in the group to which ENA #9 was administered.

### Test Example 15: Long-term Effect of ASO on Inhibition of SNCA protein

In the same manner as in Test Example 14, saline or 20 µg of ENA #9 was administered into the cerebral cortex of the SNCA E46K mice on P2, and the mouse cerebral cortex was collected one year after the administration and protein was extracted. The full-length human SNCA (hSNCA) protein level was evaluated by Western blot.

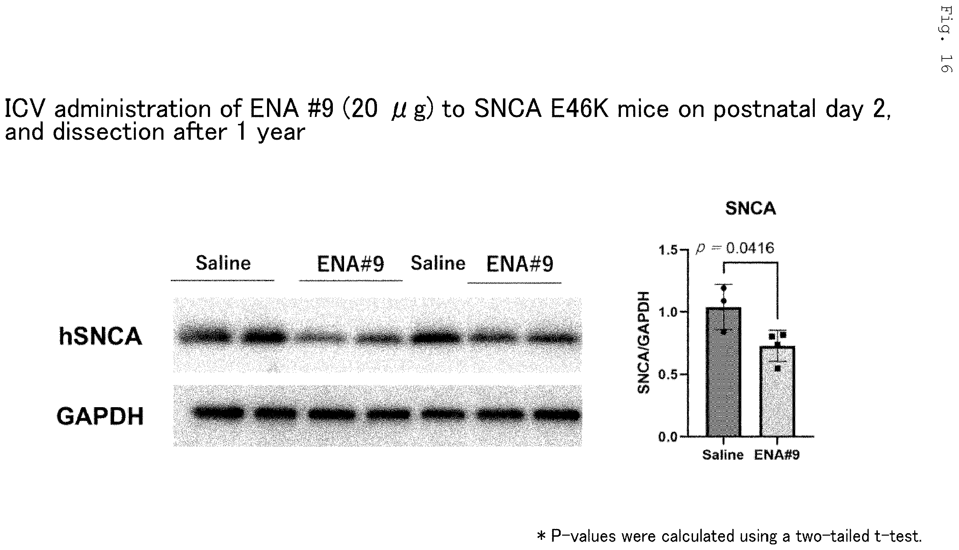

Fig. 16 shows the results. Compared to the group to which saline was administered, the hSNCA protein level was significantly decreased in the mouse cerebral cortex in the group to which ENA #9 was administered.

### Test Example 16: Therapeutic Effect of ASO on SNCA E46K Mice

20 µg of ENA #9 (n=5) or saline (n=6) was administered to homozygous SNCA E46K mice on postnatal day 2, and grip strength was measured every two weeks from 6 weeks of age to 14 weeks of age. In addition, a balance beam test was performed three times at 12 weeks of age.

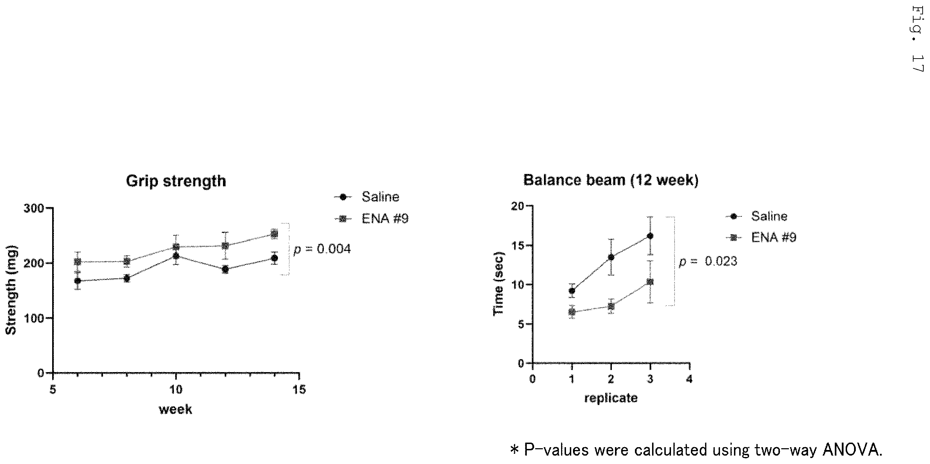

Fig. 17 shows the results. An increase in grip strength was observed in the group to which ENA #9 was administered compared to the group to which saline was administered. Furthermore, the time to reach the goal was shortened in the group to which ENA #9 was administered compared to the group to which saline was administered.

## Claims

1. A single-stranded polynucleotide comprising:
a base sequence A complementary to at least a part of an SNCA gene sequence,
wherein the base sequence A comprises a base sequence a1 complementary to the base sequence set forth in SEQ ID NO: 1 (5'-CATGGTGTGGCAAC) and/or a base sequence a2 complementary to the base sequence set forth in SEQ ID NO: 2 (5'-AGGTAAGCTCCATTGTGC) .

2. The single-stranded polynucleotide according to claim 1, wherein the base sequence A has a length of 16 or more bases.

3. The single-stranded polynucleotide according to claim 1, wherein the base sequence A has a length of 18 or more bases.

4. The single-stranded polynucleotide according to claim 1, wherein the base sequence A comprises the base sequence a1.

5. The single-stranded polynucleotide according to claim 4, wherein the base sequence A comprises, as a sequence comprising the base sequence a1, a base sequence a1' complementary to the base sequence set forth in SEQ ID NO: 38 (5'-GCATGGTGTGGCAAC).

6. The single-stranded polynucleotide according to claim 4, wherein the base sequence A comprises, as a sequence comprising the base sequence a1, a base sequence a11 complementary to a base sequence having a length of 18 or more bases within the base sequence set forth in SEQ ID NO: 3 (5'-GGTGCATGGTGTGGCAACAGGT) .

7. The single-stranded polynucleotide according to claim 4, wherein the base sequence A comprises, as a sequence comprising the base sequence a1, a base sequence a111 complementary to a base sequence having a length of 18 or more bases within the base sequence set forth in SEQ ID NO: 4 (5'-GGTGCATGGTGTGGCAACAGG) .

8. The single-stranded polynucleotide according to claim 1, wherein the base sequence A has a length of 50 or less bases.

9. The single-stranded polynucleotide according to claim 1, comprising a modified nucleotide.

10. The single-stranded polynucleotide according to claim 1, consisting of the base sequence set forth in SEQ ID NO: 14 or 32,
wherein in the base sequence, C and T are 2'-O,4'-C-ethylene nucleosides (ENA), G and A are 2'-O-methylated nucleosides (2'OMe), and all internucleoside linkages are phosphorothioate linkages.

11. A double-stranded polynucleotide comprising:
an active strand consisting of the single-stranded polynucleotide according to any one of claims 1 to 10; and
a carrier strand complementary thereto.

12. A polynucleotide comprising a coding sequence for the single-stranded polynucleotide according to any one of claims 1 to 10.

13. The polynucleotide according to claim 12, which is an expression vector for the single-stranded polynucleotide.

14. A cell comprising at least one selected from the group consisting of the single-stranded polynucleotide according to any one of claims 1 to 10, the double-stranded polynucleotide according to claim 11, and the polynucleotide according to claim 12.

15. An exon-skipping agent comprising at least one selected from the group consisting of the single-stranded polynucleotide according to any one of claims 1 to 10, the double-stranded polynucleotide according to claim 11, the polynucleotide according to claim 12, and the cell according to claim 14.

16. A pharmaceutical comprising at least one selected from the group consisting of the single-stranded polynucleotide according to any one of claims 1 to 10, the double-stranded polynucleotide according to claim 11, the polynucleotide according to claim 12, and the cell according to claim 14.

17. The pharmaceutical according to claim 16, which is for preventing or treating synucleinopathy.

18. A reagent comprising at least one selected from the group consisting of the single-stranded polynucleotide according to any one of claims 1 to 10, the double-stranded polynucleotide according to claim 11, the polynucleotide according to claim 12, and the cell according to claim 14.
